(19) Europäisches Patentamt
European Patent Office
Office européen des brevets

(11) Veröffentlichungsnummer: **0 532 996 A1**

# (12) EUROPÄISCHE PATENTANMELDUNG

(21) Anmeldenummer: **92115158.5**

(22) Anmeldetag: **04.09.92**

(51) Int. Cl.[5]: **C07D 231/22**, C07C 69/72, A01N 43/56

(30) Priorität: **17.09.91 DE 4130833**

(43) Veröffentlichungstag der Anmeldung: **24.03.93 Patentblatt 93/12**

(84) Benannte Vertragsstaaten: **BE CH DE ES FR GB IT LI NL**

(71) Anmelder: **BAYER AG**

**W-5090 Leverkusen 1 Bayerwerk(DE)**

(72) Erfinder: **Müller, Peter, Dr.**
**Talstrasse 54**
**W-4018 Langenfeld(DE)**
Erfinder: **Wolf, Hilmar, Dr.**
**Zum Bräuhaus 14**
**W-4018 Langenfeld(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

## (54) Diarylpyrazolinone.

(57) Die Erfindung betrifft neue Diarylpyrazolinone der allgemeinen Formel (I)

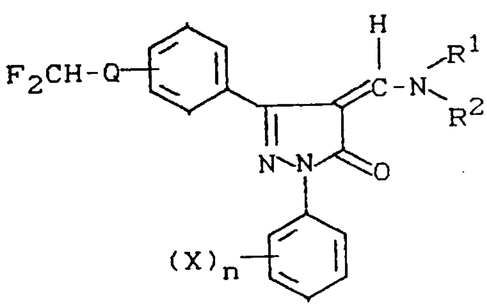

(I)

in welcher n, Q, X, $R^1$ und $R^2$ die in der Beschreibung angegebenen Bedeutungen haben, zwei Verfahren zu ihrer Herstellung, diverse neue Zwischenprodukte, sowie ihre Verwendung als Herbizide.

EP 0 532 996 A1

Die Erfindung betrifft neue Diarylpyrazolinone, zwei Verfahren zu ihrer Herstellung, diverse neue Zwischenprodukte sowie ihre Verwendung als Herbizide.

Es ist bereits bekannt, daß bestimmte Pyrazolin-5-on-Derivate, wie z.B. 5-(3-Methoxyphenyl)-4-methylaminomethylen-2-phenyl-2,4-dihydro-3H-pyrazol-3-on, herbizide Eigenschaften aufweisen (vgl. EP-A 274642). Die herbizide Wirkung der bisher bekannten Pyrazolin-5-on-Derivate ist jedoch nicht immer ganz zufriedenstellend.

Es wurden nun neue Diarylpyrazolinone der allgemeinen Formel (I) gefunden,

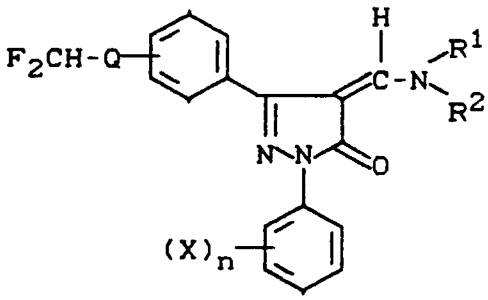

(I)

in welcher

n für die Zahlen 0, 1, 2 oder 3 steht,

Q für Sauerstoff oder Schwefel steht,

$R^1$ für Wasserstoff oder $C_1$-$C_4$-Alkyl steht,

$R^2$ für Wasserstoff, Hydroxy, Amino, oder für einen jeweils gegebenenfalls durch Halogen substituierten Rest der Reihe $C_1$-$C_6$-Alkyl, $C_2$-$C_6$-Alkenyl, $C_2$-$C_6$-Alkinyl, $C_1$-$C_6$-Hydroxyalkyl, $C_1$-$C_4$-Alkoxy-$C_1$-$C_4$-alkyl, $C_3$-$C_6$-Cycloalkyl-$C_1$-$C_4$-alkyl, Phenyl-$C_1$-$C_4$-alkyl, $C_1$-$C_6$-Alkoxy, $C_3$-$C_6$-Alkenyloxy, Phenyl-$C_1$-$C_2$-alkoxy, $C_1$-$C_6$-Alkylamino, $C_1$-$C_4$-Alkylcarbonylamino oder Di-($C_1$-$C_4$-alkyl)-amino steht, und

X für Wasserstoff, Halogen oder für einen jeweils gegebenenfalls durch Halogen substituierten Rest der Reihe $C_1$-$C_4$-Alkyl oder $C_1$-$C_4$-Alkoxy steht, wobei folgende Verbindungen - bekannt aus DE-OS 3941240, S. 44, 45, 95 und 96 - durch Disclaimer ausgenommen sind:

2-Phenyl-, 2-(4-Fluorphenyl)- und 2-(4-Chlorphenyl)-5-(2-difluormethylthiophenyl)-4-(N-hydroxymethylaminomethylen)-2,4-dihydro-3H-pyrazol-3-on, 2-Phenyl-, 2-(4-Fluorphenyl)- und 2-(4-Chlorphenyl)-5-(3-difluormethylthiophenyl)-4-(N-hydroxymethylaminomethylen)-2,4-dihydro-3H-pyrazol-3-on, 2-Phenyl-, 2-(4-Fluorphenyl)- und 2-(4-Chlorphenyl)-5-(2-difluormethylthiophenyl)-4-dimethylaminomethylen-2,4-dihydro-3H-pyrazol-3-on sowie 2-Phenyl-, 2-(4-Fluorphenyl)- und 2-(4-Chlorphenyl)-5-(3-difluormethylthiophenyl)-4-dimethylaminomethylen-2,4-dihydro-3H-pyrazol-3-on.

Die Verbindungen der Formel (I) können gegebenenfalls in verschiedenen stereoisomeren oder tautomeren Formen vorliegen. Die Erfindung betrifft sowohl die reinen Isomeren als auch die Gemische dieser Isomeren. Im nachfolgenden wird der Einfachheit halber stets von den Verbindungen der Formel (I) gesprochen, womit sowohl die reinen Isomeren als auch die verschiedenen möglichen Gemische dieser Isomeren gemeint sind.

Man erhält die neuen Verbindungen der allgemeinen Formel (I), wenn man

(a) für den Fall, daß in der Formel (I) $R^1$ und $R^2$ für Methyl stehen sowie n, Q und X die oben angegebenen Bedeutungen haben,

Pyrazolinone der allgemeinen Formel (II),

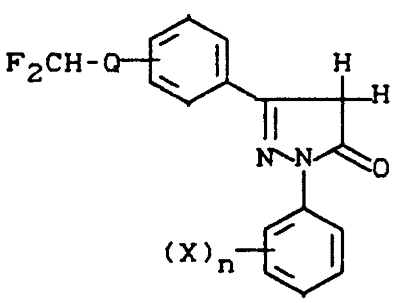

(II)

in welcher
n, Q und X die oben angegebenen Bedeutungen haben,
mit Dimethylformamid-acetalen der allgemeinen Formel (III)

$(CH_3)_2N\text{-}CH(OR)_2$ (III)

in welcher
R für $C_1$-$C_4$-Alkyl oder Benzyl steht,
gegebenenfalls in Gegenwart eines Verdünnungsmittels umsetzt, oder wenn man
(b) Diarylpyrazolinone der allgemeinen Formel (Ia),

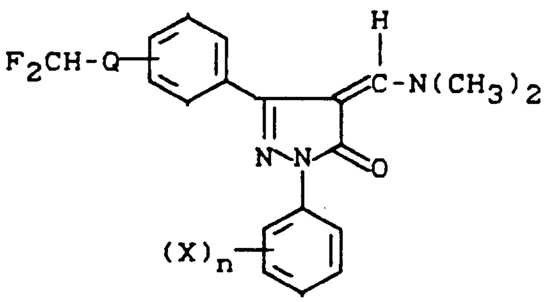
(Ia)

in welcher
n, Q und X die oben angegebenen Bedeutungen haben,
mit Aminen der allgemeinen Formel (IV),

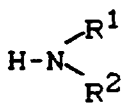
(IV)

in welcher
$R^1$ und $R^2$ die oben angegebenen Bedeutungen haben,
gegebenenfalls in Gegenwart eines Verdünnungsmittels umsetzt.

Die erfindungsgemäßen Diarylpyrazolinone der allgemeinen Formel (I) zeichnen sich durch starke herbizide Wirksamkeit aus.

Überraschenderweise zeigen die neuen Verbindungen der allgemeinen Formel (I) erheblich bessere herbizide Wirkung als die vorbekannten Pyrazolin-5-on-Derivate, welche nach Struktur und Wirkprofil vergleichbare Stoffe sind.

Gegenstand der Erfindung sind vorzugsweise Verbindungen der Formel (I), in welcher
n für die Zahlen 0, 1 oder 2 steht,
Q für Sauerstoff oder Schwefel steht,
$R^1$ für Wasserstoff, Methyl oder Ethyl steht,
$R^2$ für Wasserstoff, Hydroxy, Amino, oder für einen jeweils gegebenenfalls durch Fluor und/oder Chlor substituierten Rest der Reihe $C_1$-$C_5$-Alkyl, $C_3$-$C_5$-Alkenyl, $C_3$-$C_5$-Alkinyl, $C_1$-$C_5$-Hydroxyalkyl, $C_1$-$C_2$-Alkoxy-$C_1$-$C_2$-alkyl, $C_1$-$C_4$-Alkoxy oder $C_3$-$C_4$-Alkenyloxy;
oder für einen gegebenenfalls durch Fluor, Chlor und/oder Brom substituierten Rest der Reihe $C_3$-$C_6$-Cycloalkyl-$C_1$-$C_2$-alkyl, Phenyl-$C_1$-$C_2$-alkyl, Phenyl-$C_1$-$C_2$-alkoxy, $C_1$-$C_4$-Alkylamino, $C_1$-$C_4$-Alkylcarbonylamino oder Dimethylamino steht, und
X für Wasserstoff, Fluor, Chlor, Brom oder für einen jeweils gegebenenfalls durch Fluor und/oder Chlor substituierten Rest der Reihe Methyl, Ethyl, Methoxy oder Ethoxy steht, mit Ausnahme der durch Disclaimer ausgenommenen Verbindungen.

Die Erfindung betrifft insbesondere Verbindungen der Formel (I), in welcher
n für die Zahlen 0, 1 oder 2 steht,
Q für Sauerstoff oder Schwefel steht,
$R^1$ für Wasserstoff oder Methyl steht,
$R^2$ für Wasserstoff, Hydroxy, Amino, oder für einen jeweils gegebenenfalls durch Fluor substituierten

Rest der Reihe Methyl, Ethyl, Propyl, Isopropyl, Butyl, Isobutyl, sec-Butyl oder tert-Butyl, für Allyl, Propargyl, 1-Methyl-propargyl, 1,1-Dimethylpropargyl, Hydroxyethyl, Hydroxypropyl, Methoxyethyl, Ethoxyethyl, Cyclopropylmethyl, Cyclohexylmethyl, Cyclohexylethyl, Phenylmethyl, Phenylethyl, für Methoxy, Ethoxy, Propoxy, Allyloxy, Benzyloxy, für Methylamino, Dimethylamino, Acetylamino, Propionylamino, Butyrylamino oder Isobutyrylamino steht, und

X für Wasserstoff, Fluor, Chlor oder Trifluormethyl steht,

mit Ausnahme der durch Disclaimer ausgenommenen Verbindungen.

Verwendet man beispielsweise 1-(4-Fluor-phenyl)-3-(3-difluormethoxyphenyl)-pyrazolin-5-on und Dimethylformamiddimethylacetal als Ausgangsstoffe, so kann der Reaktionsablauf beim erfindungsgemäßen Verfahren (a) durch das folgende Formelschema skizziert werden:

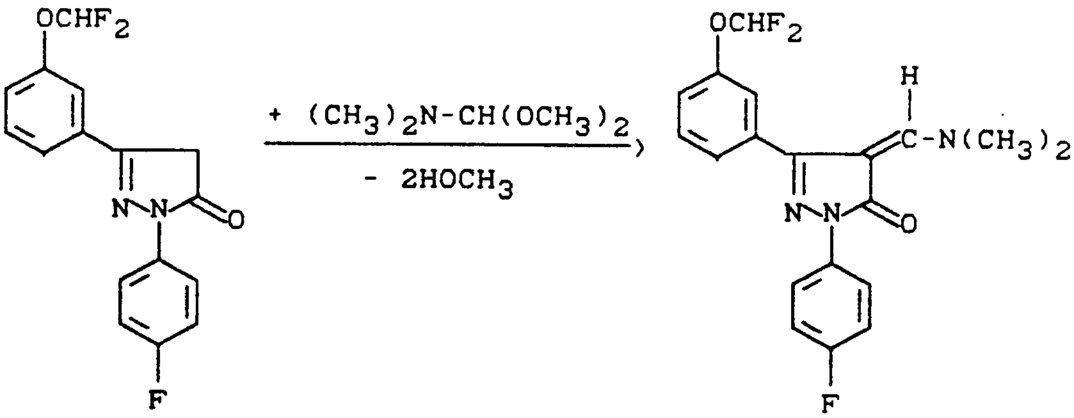


Verwendet man beispielsweise 1-(2-Chlorphenyl)-3-(4-difluormethylthiophenyl)-4-dimethylaminomethylen-pyrazolin-5-on und O-Methyl-hydroxylamin als Ausgangsstoffe, so kann der Reaktionsablauf beim erfindungsgemäßen Verfahren (b) durch das folgende Formelschema skizziert werden:

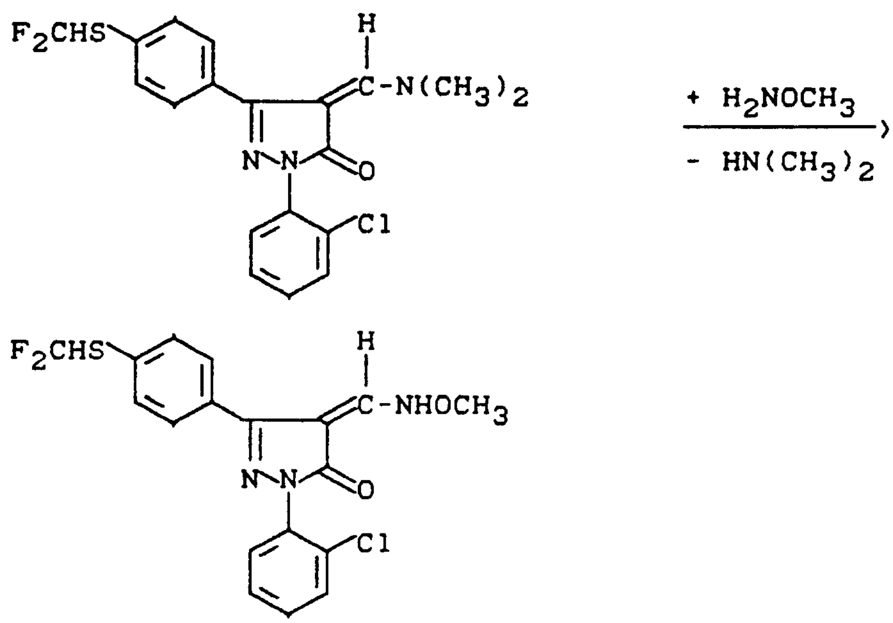


Die beim erfindungsgemäßen Verfahren (a) zur Herstellung von Verbindungen der Formel (I) als Ausgangsstoffe zu verwendenden Pyrazolinone sind durch die Formel (II) allgemein definiert.

In Formel (II) haben n, Q und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, Q und X angegeben wurden,

Die Pyrazolinone der Formel (II) sind noch nicht aus der Literatur bekannt und sind als neue Stoffe ebenfalls Gegenstand der vorliegenden Patentanmeldung.

Man erhalt die neuen Pyrazolinone der Formel (II), wenn man Aroylessigsäureester der allgemeinen Formel (V),

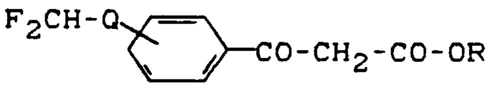

(V)

in welcher

Q für Sauerstoff oder Schwefel steht und

R für $C_1$-$C_6$-Alkyl, vorzugsweise für Methyl oder Ethyl steht,

mit Arylhydrazinen der allgemeinen Formel (VI),

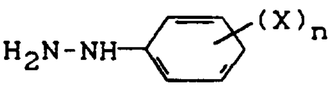

(VI)

in welcher

n für die Zahlen 0, 1, 2 oder 3 steht und

X für Wasserstoff, Halogen oder für einen jeweils gegebenenfalls durch Halogen substituierten Rest der Reihe $C_1$-$C_4$-Alkyl oder $C_1$-$C_4$-Alkoxy steht,

gegebenenfalls in Gegenwart eines Verdünnungsmittels, wie z.B, Ethanol, und gegebenenfalls in Gegenwart eines Reaktionshilfsmittels, wie z.B. Natriumacetat, bei Temperaturen zwischen -20 °C und +80 °C umsetzt und nach üblichen Methoden aufarbeitet (vgl. die Herstellungsbeispiele).

Die Arylhydrazine der Formel (VI) sind bekannt und/oder können nach an sich bekannten Verfahren hergestellt werden (vgl. US-P 4411839; DE-OS 1927924; Khim. Farm. Zh. 10 (1976), 27-31 - zitiert in Chem. Abstracts 86: 139926a).

Die Aroylessigsäureester der Formel (V) sind noch nicht aus der Literatur bekannt und sind als neue Stoffe ebenfalls Gegenstand der vorliegenden Patentanmeldung.

Man erhält die neuen Aroylessigsäureester der Formel (V), wenn man entsprechende Aroylmalonsäurediester der allgemeinen Formel (VII),

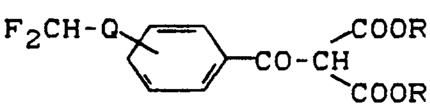

(VII)

in welcher

Q für Sauerstoff oder Schwefel steht und

R für $C_1$-$C_6$-Alkyl, vorzugsweise für Methyl oder Ethyl steht,

mit Wasser in Gegenwart eines Katalysators, wie z.B.

p-Toluolsulfonsäure, unter Rückfluß erhitzt und dann nach üblichen Methoden aufarbeitet (vgl. die Herstellungsbeispiele).

Die Aroylmalonsäurediester der Formel (VII) sind noch nicht aus der Literatur bekannt und sind als neue Stoffe ebenfalls Gegenstand der vorliegenden Patentanmeldung.

Man erhält die neuen Aroylmalonsäurediester der Formel (VII), wenn man Aroylhalogenide der allgemeinen Formel (VIII),

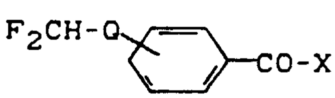

(VIII)

in welcher

Q für Sauerstoff oder Schwefel steht und

X für Halogen, insbesondere Fluor, Chlor oder Brom steht,

mit Malonsäurediestern der allgemeinen Formel (IX),

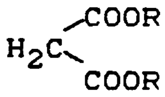 (IX)

in welcher

R für $C_1$-$C_6$-Alkyl, vorzugsweise für Methyl oder Ethyl steht,

in Gegenwart eines Verdünnungsmittels, wie z.B` Acetonitril, in Gegenwart eines Reaktionshilfsmittels, wie z.B, Magnesiumchlorid, und in Gegenwart eines Säurebindemittels, wie z.B. Triethylamin, bei Temperaturen zwischen -20 ° C und +50 ° C umsetzt und nach üblichen Methoden aufarbeitet (vgl. die Herstellungsbeispiele).

Die Aroylhalogenide der Formel (VIII) sind bekannt und/oder können nach an sich bekannten Verfahren hergestellt werden (vgl. US-P 4832879; JP 59181259 - zitiert in Chem. Abstracts 102: 113480z; DE-OS 2914915; US-P 4009208; US-P 3960945; US-P 3895036; Ukr. Khim. Zh. 47 (1981), 871-874 - zitiert in Chem. Abstracts 95: 186790x).

Die Malonsäurediester der Formel (IX) sind bekannte Synthesechemikalien.

Die beim erfindungsgemäßen Verfahren (b) zur Herstellung von Verbindungen der Formel (I) als Ausgangsstoffe zu verwendenden Diarylpyrazolinone sind durch die Formel (Ia) allgemein definiert.

In Formel (Ia) haben n, Q und X vorzugsweise bzw, insbesondere diejenigen Bedeutungen, die bereits oben im Zusammenhang mit der Beschreibung der erfindungsgemäßen Verbindungen der Formel (I) vorzugsweise bzw, als insbesondere bevorzugt für n, Q und X angegeben wurden.

Die Ausgangsstoffe der Formel (Ia) sind eine Teilmenge der erfindungsgemäßen Diarylpyrazolinone der Formel (I); sie können nach dem erfindungsgemäßen Verfahren (a) hergestellt werden.

Die beim erfindungsgemäßen Verfahren (b) weiter als Ausgangsstoffe zu verwendenden Amine sind durch die Formel (IV) allgemein definiert.

In Formel (IV) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und $R^2$ angegeben wurden.

Die Ausgangsstoffe der Formel (IV) sind bekannte Synthesechemikalien.

Die erfindungsgemäßen Verfahren (a) und (b) zur Herstellung der neuen Diarylpyrazolinone der Formel (I) werden vorzugsweise unter Verwendung von Verdünnungsmitteln durchgeführt. Als Verdünnungsmittel kommen dabei alle inerten organischen Losungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole wie Methanol, Ethanol und Isopropanol, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen Können bei dem erfindungsgemäßen Verfahren (a) und (b) in einem größeren Bereich variiert werden, Im allgemeinen arbeitet man bei Temperaturen zwischen 0 ° C und 100 ° C, vorzugsweise bei Temperaturen zwischen 10 ° C und 50 ° C.

Die erfindungsgemäßen Verfahren (a) und (b) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a) und (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden, Die Reaktionen werden im allgemeinen in einem geeigneten Verdünnungsmittel durchgeführt und das Reaktionsgemisch wird mehrere Stunden bei der jeweils erforderlichen Temperatur gerührt. Die Aufarbeitung erfolgt jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen, sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden, Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen infrage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z,B, gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z,B, nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide, Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken, Sie hängt im wesentlichen von der Art des gewünschten Effektes ab, Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

Herstellungsbeispiele:

Beispiel 1

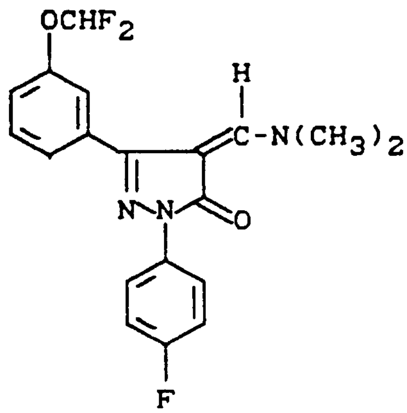

(Verfahren (a))

Eine Mischung aus 6,5 g (20 mMol) 2-(4-Fluorphenyl)-5-(3-difluormethoxyphenyl)-4-dimethylaminomethylen-2,4-dihydro-3H-pyrazol-3-on, 2,7 g (22 mMol) Dimethylformamid-dimethylacetal und 150 ml Toluol wird 2 Stunden bei 20 ° C gerührt. Dann wird im Wasserstrahlvakuum eingeengt, der Rückstand mit Petrolether verrieben und das kristallin anfallende Produkt durch Abfiltrieren isoliert.

Man erhält 6,9 g (92% der Theorie) 2-(4-Fluorphenyl)-5-(3-difluormethoxyphenyl)-4-dimethylaminomethylen-2,4-dihydro-3H-pyrazol-3-on vom Schmelzpunkt 103 ° C.

Beispiel 2

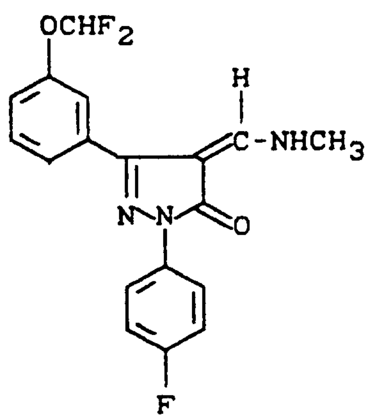

(Verfahren (b))

Eine Mischung aus 2,8 g (7,5 mMol) 2-(4-Fluorphenyl)-5-(3-difluormethoxyphenyl)-4—methylaminomethylen-2,4-dihydro-3H-pyrazol-3-on, 50 ml Methanol und 3 ml einer 30%igen wäßrigen Methylamin-Lösung (10 mMol $H_2NCH_3$),wird 3 Stunden bei 20 ° C gerührt. Dann wird im Wasserstrahlvakuum eingeengt, der Rückstand mit Petrolether verrieben und das kristallin angefallende Produkt durch Abfiltrieren isoliert.

Man erhält 2,4 g (88,5% der Theorie) 2-(4-Fluorphenyl)-5-(3-difluormethoxyphenyl)-4-methylaminomethylen-2,4-dihydro-3H-pyrazol-3-on vom Schmelzpunkt 187 ° C.

Analog zu den Herstellungsbeispielen 1 und 2 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

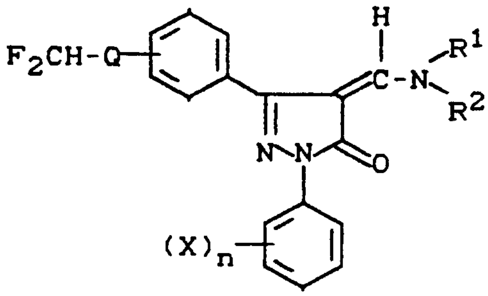

(I)

Tabelle 1: Beispiele für die Verbindungen der Formel (I)

| Bsp.-Nr. | n | (Position-) Q | $R^1$ | $R^2$ | (Position-) X | Schmelzpunkt (°C) |
|---|---|---|---|---|---|---|
| 3 | 1 | (2-)S | H | $CH_3$ | (4-)F | |
| 4 | 2 | (2-)S | $CH_3$ | $CH_3$ | (2,4-)$F_2$ | 122 |
| 5 | 0 | (3-)O | $CH_3$ | OH | - | 111 |
| 6 | 1 | (2-)S | $CH_3$ | $CH_3$ | (4-)$CF_3$ | 93 |
| 7 | 1 | (2-)S | H | $CH_3$ | (4-)$CF_3$ | 139 |
| 8 | 1 | (2-)S | H | $NHCH_3$ | (4-)F | 125 |
| 9 | 1 | (2-)S | H | $N(CH_3)_2$ | (4-)F | 108 |
| 10 | 1 | (2-)S | $CH_3$ | $CH_3$ | (3-)Cl | 104 |
| 11 | 0 | (2-)S | H | $CH_3$ | - | 116 |
| 12 | 1 | (2-)S | H | $NHCH_3$ | (4-)$CF_3$ | 137 |
| 13 | 1 | (2-)S | H | $N(CH_3)_2$ | (4-)$CF_3$ | 107 |
| 14 | 1 | (2-)S | $CH_3$ | OH | (4-)$CF_3$ | 118 |
| 15 | 2 | (2-)S | H | $CH_3$ | (2,4-)$F_2$ | 75 |
| 16 | 0 | (2-)S | H | $NHCH_3$ | - | |
| 17 | 0 | (2-)S | H | $N(CH_3)_2$ | - | |
| 18 | 1 | (3-)O | H | $CH_3$ | (2-)F | 126 |
| 19 | 1 | (2-)S | H | $CH_3$ | (3-)Cl | 138 |
| 20 | 1 | (2-)S | H | $CH_3$ | (4-)Cl | 145 |
| 21 | 1 | (3-)O | $CH_3$ | OH | (2-)F | 78 |
| 22 | 2 | (2-)S | $CH_3$ | OH | (2,4-)$F_2$ | 141 |

Tabelle 1 - Fortsetzung

| Bsp.-Nr. | n | (Position-) Q | $R^1$ | $R^2$ | (Position-) X | Schmelzpunkt (°C) |
|---|---|---|---|---|---|---|
| 23 | 1 | (2-)S | H | $CH_3$ | (2-)F | 127 |
| 24 | 1 | (2-)S | H | $NHCH_3$ | (3-)Cl | 88 |
| 25 | 0 | (3-)O | H | $CH_3$ | - | 137 |
| 26 | 1 | (2-)S | $CH_3$ | OH | (3-)Cl | 147 |
| 27 | 0 | (3-)S | H | $CH_3$ | - | 79 |
| 28 | 1 | (3-)S | $CH_3$ | $CH_3$ | (3-)Cl | 132 |
| 29 | 1 | (3-)S | $CH_3$ | $CH_3$ | (4-)$CF_3$ | 121 |
| 30 | 1 | (3-)S | $CH_3$ | $CH_3$ | (3-)F | 114 |
| 31 | 1 | (3-)S | H | $N(CH_3)_2$ | (4-)F | |
| 32 | 1 | (3-)S | H | $CH_3$ | (4-)F | 105 |
| 33 | 1 | (2-)S | $CH_3$ | OH | (2-)F | |
| 34 | 1 | (3-)O | $CH_3$ | OH | (4-)F | |
| 35 | 1 | (3-)S | $CH_3$ | OH | (3-)Cl | 105 |
| 36 | 1 | (3-)S | $CH_3$ | OH | (3-)F | 58 |
| 37 | 1 | (3-)S | H | $CH_3$ | (3-)Cl | 97 |
| 38 | 1 | (3-)S | H | $CH_3$ | (3-)F | 92 |
| 39 | 1 | (2-)S | H | $CH_3$<br>\|<br>-C-C≡CH<br>\|<br>$CH_3$ | (4-)F | 99 |
| 40 | 1 | (3-)S | H | $CH_3$ | (4-)$CF_3$ | 127 |

Tabelle 1 - Fortsetzung

| Bsp.-Nr. | n | (Position-) Q | $R^1$ | $R^2$ | (Position-) X | Schmelzpunkt (°C) |
|---|---|---|---|---|---|---|
| 41 | 1 | (3-)S | $CH_3$ | OH | (4-)$CF_3$ | 135 |
| 42 | 1 | (3-)S | H | $NHCH_3$ | (3-)Cl | |
| 43 | 1 | (2-)S | H | 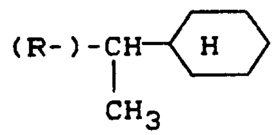 | (4-)F | 49 |
| 44 | 1 | (2-)S | H | $-CH_2C(CH_3)_3$ | (4-)F | 105 |
| 45 | 1 | (2-)S | H | 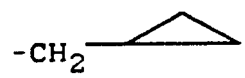 | (4-)F | 84 |
| 46 | 1 | (2-)S | H | $-CH_2-C{\equiv}CH$ | (4-)F | 158 |
| 47 | 1 | (2-)S | H | $C_4H_9$ | (4-)F | |
| 48 | 1 | (2-)S | H | 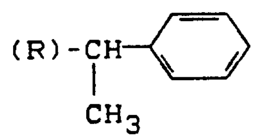 | (4-)F | 58 |
| 49 | 1 | (2-)S | H | 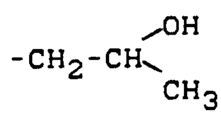 | (4-)F | 127 |
| 50 | 1 | (2-)S | H | $OCH_3$ | (4-)F | 46 |
| 51 | 1 | (2-)S | H | 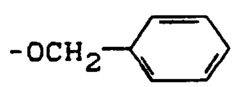 | (4-)F | |
| 52 | 1 | (2-)S | H | $-CH_2CH_2OC_2H_5$ | (4-)F | 61 |
| 53 | 1 | (2-)S | H | 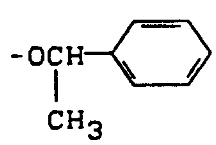 | (4-)F | |

Tabelle 1 - Fortsetzung

| Bsp.-Nr. | n | (Position-) Q | $R^1$ | $R^2$ | (Position-) X | Schmelzpunkt (°C) |
|---|---|---|---|---|---|---|
| 54 | 1 | (2-)S | H | 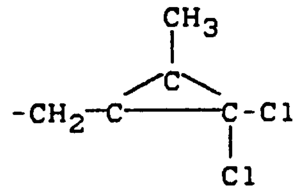 | (4-)F | 113 |
| 55 | 1 | (2-)S | H | $-CH_2CH_2OCH_3$ | (4-)F | 81 |
| 56 | 1 | (2-)S | H | 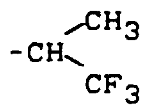 | (4-)F | 45 |
| 57 | 1 | (2-)S | H | 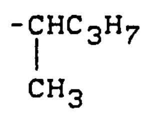 | (4-)F | 62 |
| 58 | 1 | (2-)S | H | $-OCH_2CH=CH_2$ | (4-)F | 48 |
| 59 | 1 | (2-)S | H | H | (4-)F | |
| 60 | 1 | (2-)S | H | $-C(CH_3)_3$ | (4-)F | |
| 61 | 1 | (2-)S | H | $-CH(CH_3)_2$ | (4-)F | 99 |
| 62 | 1 | (2-)S | H | $-CH_2CH(CH_3)_2$ | (4-)F | |
| 63 | 1 | (2-)O | $CH_3$ | $CH_3$ | (4-)F | 120 |
| 64 | 1 | (2-)S | H | $-CO-CH_3$ | (4-)F | |
| 65 | 1 | (2-)S | H | $-CO-CH(CH_3)_2$ | (4-)F | 80 |
| 66 | 1 | (2-)S | H | $-CO-C_2H_5$ | (4-)F | 96 |
| 67 | 1 | (2-)O | $CH_3$ | OH | (4-)F | 154 |
| 68 | 1 | (2-)O | H | $CH_3$ | (4-)F | 193 |
| 69 | 0 | (3-)O | $CH_3$ | $CH_3$ | - | 136 |

Tabelle 1 - Fortsetzung

| Bsp.-Nr. | n | (Position-) Q | $R^1$ | $R^2$ | (Position-) X | Schmelzpunkt (°C) |
|---|---|---|---|---|---|---|
| 70 | 1 | (3-)O | $CH_3$ | OH | (4-)F | 146 |
| 71 | 1 | (2-)S | $CH_3$ | OH | (2-)F | 124 |
| 72 | 0 | (3-)O | $CH_3$ | H | - | 137 |
| 73 | 1 | (2-)S | $CH_3$ | H | (2-)F | 127 |
| 74 | 1 | (3-)O | $CH_3$ | OH | (2-)F | 78 |
| 75 | 1 | (3-)O | $CH_3$ | H | (2-)F | 126 |

Ausganasstoffe der Formel (II):

Beispiel (II-1)

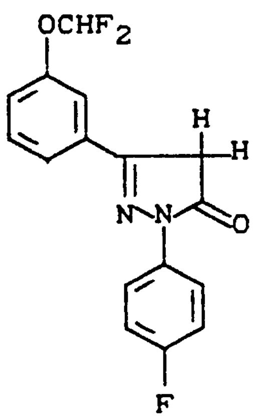

Zu einer Lösung von 4,06 g (25 mMol) 4-Fluorphenylhydrazin-hydrochlorid in 100 ml Ethanol werden 6,5 g (25 mMol) (3-Difluormethoxybenzoyl)-essigsäureethylester und 2,05 g (25 mMol) Natriumacetat gegeben und das Reaktionsgemisch wird 20 Stunden bei 20° C gerührt. Dann wird im Wasserstrahlvakuum eingeengt, der Rückstand mit Petrolether verrührt, das kristalline Produkt durch Absaugen isoliert, mit Wasser gewaschen und getrocknet.

Man erhalt 7,4 g (92% der Theorie) 2-(4-Fluorphenyl)-5-(3-difluormethoxyphenyl)-2,4-dihydro-3H-pyrazol-3-on vom Schmelzpunkt 114° C.

Analog Beispiel (II-1) können auch die in der nachstehenden Tabelle 2 aufgeführten Verbindungen der Formel (II) hergestellt werden.

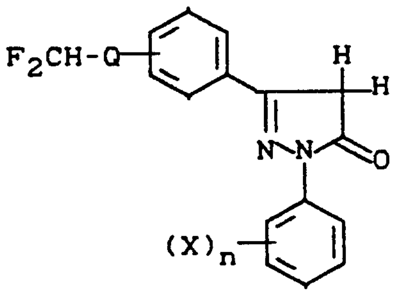

Tabelle 2

| Beispiele für die Verbindungen der Formel (II) | | | | |
|---|---|---|---|---|
| Bsp.-Nr. | n | (Position-) Q | (Position-) X | Schmelzpunkt (° C) |
| (II-2) | 1 | (2-)S | (4-)F | |
| (II-3) | 1 | (3-)S | (4-)F | 78 |
| (II-4) | 1 | (2-)O | (4-)F | |
| (II-5) | 1 | (2-)S | (4-)Cl | |
| (II-6) | 2 | (2-)S | (2,4-)$F_2$ | |
| (II-7) | 1 | (2-)S | (3-)Cl | |
| (II-8) | 1 | (3-)S | (3-)Cl | |
| (II-9) | 1 | (3-)S | (4-)$CF_3$ | |
| (II-10) | 1 | (2-)S | (2-)F | |
| (II-11) | 1 | (3-)O | (2-)F | |
| (II-12) | 1 | (3-)O | (4-)F | |
| (II-13) | 1 | (3-)O | H | |

Ausgangsstoffe der Formel (V):

Beispiel (V-1)

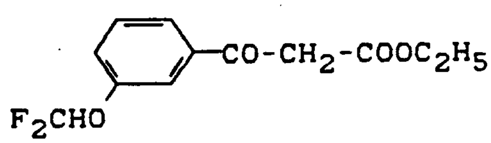

Eine Mischung aus 91,5 g (0,28 Mol) (3-Difluormethoxybenzoyl)-malonsäurediethylester, 300 ml Wasser und 3 g p-Toluolsulfonsäure wird 8 Stunden unter Rückfluß erhitzt. Nach dem Abkühlen wird mit Methylenchlorid extrahiert, die organische Phase mit gesättigter Natriumhydrogencarbonatlösung verrührt, mit Natriumsulfat getrocknet und filtriert. Das Filtrat wird im Wasserstrahlvakuum eingeengt und der Rückstand im Ölpumpenvakuum destilliert.

Man erhält 21,2 g (30% der Theorie) (3-Difluormethoxybenzoyl)-essigsäureethylester vom Siedepunkt 118° C (bei 0,01 Torr).

Ausgangsstoffe der Formel (VII):

Beispiel (VII-1)

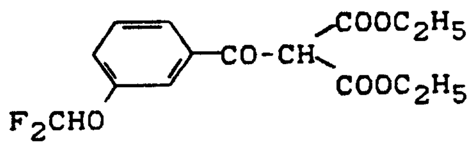

Zu 150 ml Acetonitril werden 27,4 g (0,29 Mol) Magnesiumchlorid portionsweise gegeben, wobei durch Außenkühlung mit Eis/Wasser die Innentemperatur unterhalb von 25° C gehalten wird. Nach Abkühlen auf -10° C werden nacheinander 46,1 g (0,29 Mol) Malonsäurediethylester und 58,1 g (0,58 Mol) Triethylamin zugetropft. Nach Rühren der Mischung für weitere 30 Minuten bei -10° C werden 59,5 g (0,29 Mol) 3-Difluormethoxy-benzoylchlorid, gelöst in 120 ml Acetonitril zugetropft und das Gemisch wird 12 Stunden bei 20° C gerührt. Nach Zugabe von 250 ml 5N-Salzsäure wird mit Methylenchlorid extrahiert, die organische Phase mit Natriumsulfat getrocknet und filtriert. Vom Filtrat wird das Lösungsmittel im Wasserstrahlvakuum sorgfältig abdestilliert.

Man erhält 92,4 g (97% der Theorie) (3-(Difluormethoxybenzoyl)-malonsäurediethylester als öligen Rückstand mit Brechungsindex $n_D^{24}$ : 1,4629.

Anwendungsbeispiele:

In den folgenden Anwendungsbeispielen wird die nachstehend aufgeführte Verbindung als Vergleichssubstanz herangezogen:

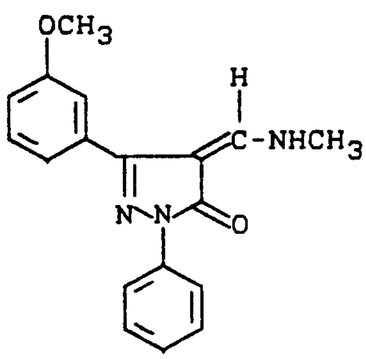

(A)

5-(3-Methoxyphenyl)-4-methylaminomethylen-2-phenyl-2,4-dihydro-3H-pyrazol-3-on
(bekannt aus EP-A 274642).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Nach drei Wochen wird der Schädigungsgrund der Pflanzen bonitiert in% Schädigung im Vergleich zur Entwicklung der unbehandelten Kontrolle.

Es bedeuten:

O % = keine Wirkung (wie unbehandelte Kontrolle)
100 % = totale Vernichtung
In diesem Test zeigen beispielsweise die Verbindungen gemäß den Herstellungsbeispielen 3, 5, 7, 8, 10, 11, 14, 15, 17, 19, 20, 26, 27, 32, 33, 36, 38, 40, 52, 55, 59 und 67 bei guter Kulturpflanzen-Verträglichkeit starke Wirkung gegen Unkräuter.

Beispiel B

Pre-emergence-Test

Lösungsmittel: 5 Gewichtsteile Aceton
Emulgator: 1 Gewichtsteil Alkylarylpolyglykolether
Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
O % = keine Wirkung (wie unbehandelte Kontrolle)
100 % = totale Vernichtung
Eine deutliche Überlegenheit in der Wirksamkeit gegenüber dem Stand der Technik zeigen in diesem Test z.B. die Verbindungen gemäß folgender Herstellungsbeispiele: 7 und 15.

## Patentansprüche

**1.** Diarylpyrazolinone der allgemeinen Formel (I)

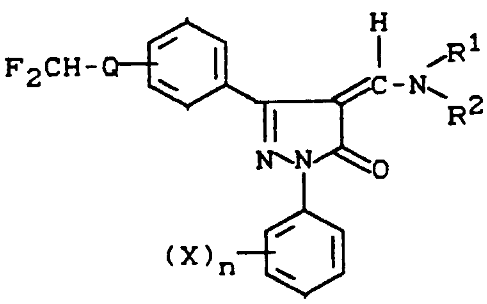

(I)

dadurch gekennzeichnet, daß

n für die Zahlen 0, 1, 2 oder 3 steht,

Q für Sauerstoff oder Schwefel steht,

$R^1$ für Wasserstoff oder $C_1$-$C_4$-Alkyl steht,

$R^2$ für Wasserstoff, Hydroxy, Amino, oder für einen jeweils gegebenenfalls durch Halogen substituierten Rest der Reihe $C_1$-$C_6$-Alkyl, $C_2$-$C_6$-Alkenyl, $C_2$-$C_6$-Alkinyl, $C_1$-$C_6$-Hydroxyalkyl, $C_1$-$C_4$-Alkoxy-$C_1$-$C_4$-alkyl, $C_3$-$C_6$-Cycloalkyl-$C_1$-$C_4$-alkyl, Phenyl-$C_1$-$C_4$-alkyl, $C_1$-$C_6$-Alkoxy, $C_3$-$C_6$-Alkenyloxy, Phenyl-$C_1$-$C_2$-alkoxy, $C_1$-$C_6$-Alkylamino, $C_1$-$C_4$-Alkylcarbonylamino oder Di-($C_1$-$C_4$-alkyl)-amino steht, und

X für Wasserstoff, Halogen oder für einen jeweils gegebenenfalls durch Halogen substituierten Rest der Reihe $C_1$-$C_4$-Alkyl oder $C_1$-$C_4$-Alkoxy steht, ausgenommen die Verbindungen:

2-Phenyl-, 2-(4-Fluorphenyl)- und 2-(4-Chlorphenyl)-5-(2-difluormethylthiophenyl)-4-(N-hydroxy-methylaminomethylen)-2,4-dihydro-3H-pyrazol-3-on, 2-Phenyl-, 2-(4-Fluorphenyl)- und 2-(4-Chlorphenyl)-5-(3-difluormethylthiophenyl)-4-(N-hydroxy-methylaminomethylen)-2,4-dihydro-3H-pyrazol-3-on, 2-Phenyl-, 2-(4-Fluorphenyl)- und 2-(4-Chlorphenyl)-5-(2-difluormethylthiophenyl)-4-dimethylaminomethylen-2,4-dihydro-3H-pyrazol-3-on sowie 2-Phenyl-, 2-(4-Fluorphenyl)- und 2-(4-Chlorphenyl)-5-(3-difluormethylthiophenyl)-4-dimethylaminomethylen-2,4-dihydro-3H-pyrazol-3-on.

**2.** Diarylpyrazolinone der allgemeinen Formel (I) gemäß Anspruch 1, dadurch gekennzeichnet, daß

n für die Zahlen 0, 1 oder 2 steht,

Q für Sauerstoff oder Schwefel steht,

$R^1$ für Wasserstoff, Methyl oder Ethyl steht,

$R^2$ für Wasserstoff, Hydroxy, Amino, oder für einen jeweils gegebenenfalls durch Fluor und/oder Chlor substituierten Rest der Reihe $C_1$-$C_5$-Alkyl, $C_3$-$C_5$-Alkenyl, $C_3$-$C_5$-Alkinyl, $C_1$-$C_5$-Hydroxyalkyl, $C_1$-$C_2$-Alkoxy-$C_1$-$C_2$-alkyl, $C_1$-$C_4$-Alkoxy oder $C_3$-$C_4$-Alkenyloxy, oder für einen gegebenenfalls durch Fluor, Chlor und/oder Brom substituierten Rest der Reihe $C_3$-$C_6$-Cycloalkyl-$C_1$-$C_2$-alkyl, Phenyl-$C_1$-$C_2$-alkyl, Phenyl-$C_1$-$C_2$-alkoxy, $C_1$-$C_4$-Alkylamino, $C_1$-$C_4$-Alkylcarbonylamino oder Dimethylamino steht, und

X für Wasserstoff, Fluor, Chlor, Brom oder für einen jeweils gegebenenfalls durch Fluor und/oder Chlor substituierten Rest der Reihe Methyl, Ethyl, Methoxy oder Ethoxy steht, ausgenommen die Verbindungen:

2-Phenyl-, 2-(4-Fluorphenyl)- und 2-(4-Chlorphenyl)-5-(2-difluormethylthiophenyl)-4-(N-hydroxy-methylaminomethylen)-2,4-dihydro-3H-pyrazol-3-on, 2-Phenyl-, 2-(4-Fluorphenyl)- und 2-(4-Chlorphenyl)-5-(3-difluormethylthiophenyl)-4-(N-hydroxy-methylaminomethylen)-2,4-dihydro-3H-pyrazol-3-on, 2-Phenyl-, 2-(4-Fluorphenyl)- und 2-(4-Chlorphenyl)-5-(2-difluormethylthiophenyl)-4-dimethylaminomethylen-2,4-dihydro-3H-pyrazol-3-on sowie 2-Phenyl-, 2-(4-Fluorphenyl)- und 2-(4-Chlorphenyl)-5-(3-difluormethylthiophenyl)-4-dimethylaminomethylen-2,4-dihydro-3H-pyrazol-3-on.

**3.** Diarylpyrazolinone der allgemeinen Formel (I) gemäß Anspruch 1, dadurch gekennzeichnet, daß

n für die Zahlen 0, 1 oder 2 steht,

Q für Sauerstoff oder Schwefel steht,

$R^1$ für Wasserstoff oder Methyl steht,

$R^2$ für Wasserstoff, Hydroxy, Amino, oder für einen jeweils gegebenenfalls durch Fluor substituierten Rest der Reihe Methyl, Ethyl, Propyl, Isopropyl, Butyl, Isobutyl, sec-Butyl oder tert-Butyl, für Allyl, Propargyl, 1-Methyl-propargyl, 1,1-Dimethyl-propargyl, Hydroxyethyl, Hydroxypropyl, Methoxyethyl, Ethoxyethyl, Cyclopropylmethyl, Cyclohexylmethyl, Cyclohexylethyl, Phenylmethyl, Phenylethyl, für Methoxy, Ethoxy, Propoxy, Allyloxy, Benzyloxy, für Methylamino, Dimethylamino, Acetylamino, Propionylamino, Butyrylamino oder Isobutyrylamino steht, und

X für Wasserstoff, Fluor, Chlor oder Trifluormethyl steht, ausgenommen die Verbindungen:

2-Phenyl-, 2-(4-Fluorphenyl)- und 2-(4-Chlorphenyl)-5-(2-difluormethylthiophenyl)-4-(N-hydroxy-methylaminomethylen)-2,4-dihydro-3H-pyrazol-3-on, 2-Phenyl-, 2-(4-Fluorphenyl)- und 2-(4-Chlorphenyl)-5-(3-difluormethylthiophenyl)-4-(N-hydroxy-methylaminomethylen)-2,4-dihydro-3H-pyrazol-3-on, 2-Phenyl-, 2-(4-Fluorphenyl)- und 2-(4-Chlorphenyl)-5-(2-difluormethylthiophenyl)-4-dimethylaminomethy len-2,4-dihydro-3H-pyrazol-3-on sowie 2-Phenyl-, 2-(4-Fluorphenyl)- und 2-(4-Chlorphenyl)-5-(3-difluormethylthiophenyl)-4-dimethylaminomethylen-2,4-dihydro-3H-pyrazol-3-on.

**4.** Verfahren zur Herstellung von Diarylpyrazolinonen der allgemeinen Formel (I) gemäß Anspruch 1,

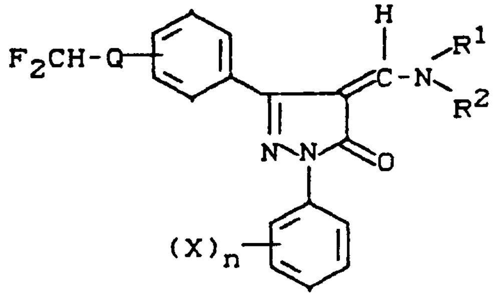

(I)

in welcher

n für die Zahlen 0, 1, 2 oder 3 steht,

Q für Sauerstoff oder Schwefel steht,

$R^1$ für Wasserstoff oder $C_1$-$C_4$-Alkyl steht,

$R^2$ für Wasserstoff, Hydroxy, Amino, oder für einen jeweils gegebenenfalls durch Halogen substituierten Rest der Reihe $C_1$-$C_6$-Alkyl, $C_2$-$C_6$-Alkenyl, $C_2$-$C_6$-Alkinyl, $C_1$-$C_6$-Hydroxyalkyl, $C_1$-$C_4$-Alkoxy-$C_1$-$C_4$-alkyl, $C_3$-$C_6$-Cycloalkyl-$C_1$-$C_4$-alkyl, Phenyl-$C_1$-$C_4$-alkyl, $C_1$-$C_6$-Alkoxy, $C_3$-$C_6$-Alkenyloxy, Phenyl-$C_1$-$C_2$-alkoxy, $C_1$-$C_6$-Alkylamino, $C_1$-$C_4$-Alkylcarbonylamino oder Di-($C_1$-$C_4$-alkyl)-amino steht, und

X für Wasserstoff, Halogen oder für einen jeweils gegebenenfalls durch Halogen substituierten Rest der Reihe $C_1$-$C_4$-Alkyl oder $C_1$-$C_4$-Alkoxy steht,

wobei folgende Verbindungen durch Disclaimer ausgenommen sind:

2-Phenyl-, 2-(4-Fluorphenyl)- und 2-(4-Chlorphenyl)-5-(2-difluormethylthiophenyl)-4-(N-hydroxy-methylaminomethylen)-2,4-dihydro-3H-pyrazol-3-on, 2-Phenyl-, 2-(4-Fluorphenyl)- und 2-(4-Chlorphenyl)-5-(3-difluormethylthiophenyl)-4-(N-hydroxy-methylaminomethylen)-2,4-dihydro-3H-pyrazol-3-on, 2-Phenyl-, 2-(4-Fluorphenyl)- und 2-(4-Chlorphenyl)-5-(2-difluormethylthiophenyl)-4-dimethylaminomethylen-2,4-dihydro-3H-pyrazol-3-on sowie 2-Phenyl-, 2-(4-Fluorphenyl)- und 2-(4-Chlorphenyl)-5-(3-difluormethylthiophenyl)-4-dimethylaminomethylen-2,4-dihydro-3H-pyrazol-3-on,

dadurch gekennzeichnet, daß man

(a) für den Fall, daß in der Formel (I) $R^1$ und $R^2$ für Methyl stehen sowie n, Q und X die oben angegebenen Bedeutungen haben,

Pyrazolinone der allgemeinen Formel (II),

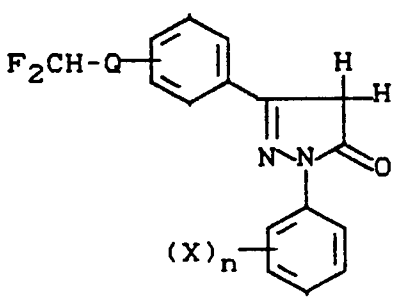

(II)

in welcher

n, Q und X die oben angegebenen Bedeutungen haben,

mit Dimethylformamid-acetalen der allgemeinen Formel (III)

$(CH_3)_2N$-$CH(OR)_2$ (III)

in welcher

R für $C_1$-$C_4$-Alkyl oder Benzyl steht,

gegebenenfalls in Gegenwart eines Verdünnungsmittels umsetzt, oder daß man

(b) Diarylpyrazolinone der allgemeinen Formel (Ia),

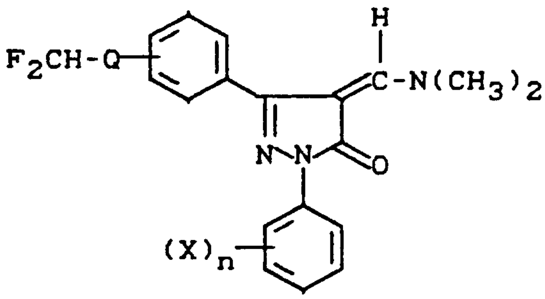

(Ia)

in welcher

n, Q und X die oben angegebenen Bedeutungen haben,

mit Aminen der allgemeinen Formel (IV),

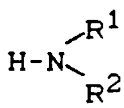

(IV)

in welcher

$R^1$ und $R^2$ die oben angegebenen Bedeutungen haben,

gegebenenfalls in Gegenwart eines Verdünnungsmittels umsetzt.

**5.** Herbizide Mittel, gekennzeichnet durch einen Gehalt an mindestens einem Diarylpyrazolinon der Formel (I) gemäß den Ansprüchen 1 bis 4.

**6.** Verfahren zur Bekämpfung von unerwünschten Pflanzen, dadurch gekennzeichnet, daß man Diarylpyrazolinone der Formel (I) gemäß den Ansprüchen 1 bis 4 auf unerwünschte Pflanzen und/oder ihren Lebensraum einwirken läßt.

**7.** Verwendung von Diarylpyrazolinonen der Formel (I) gemäß den Ansprüchen 1 bis 4 zur Bekämpfung von unerwünschten Pflanzen.

**8.** Verfahren zur Herstellung von herbiziden Mitteln, dadurch gekennzeichnet, daß man Diarylpyrazolinone der Formel (I) gemäß den Ansprüchen 1 bis 4 mit Streckmitteln und/oder oberflächenaktiven Substanzen vermischt.

**9.** Pyrazolinone der allgemeinen Formel (II)

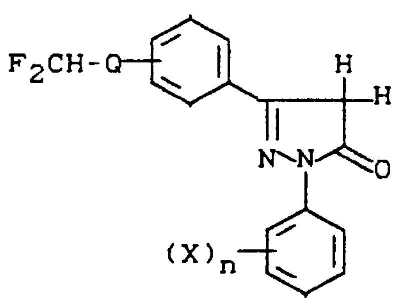

(II)

dadurch gekennzeichnet, daß

n für die Zahlen 0, 1, 2 oder 3 steht,

Q für Sauerstoff oder Schwefel steht und

X für Wasserstoff, Halogen oder für einen jeweils gegebenenfalls durch Halogen substituierten Rest der Reihe $C_1$-$C_4$-Alkyl oder $C_1$-$C_4$-Alkoxy steht.

**10.** Aroylessigsäureester der allgemeinen Formel (V),

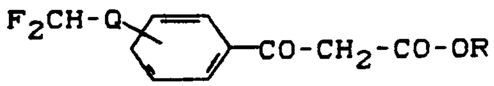

(V)

dadurch gekennzeichnet, daß

Q für Sauerstoff oder Schwefel steht und

R für $C_1$-$C_6$-Alkyl, vorzugsweise für Methyl oder Ethyl steht.

**11.** Aroylmalonsäurediester der allgemeinen Formel (VII),

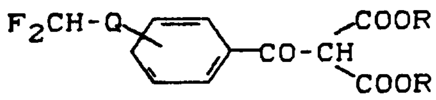

(VII)

in welcher

Q für Sauerstoff oder Schwefel steht und

R für $C_1$-$C_6$-Alkyl, vorzugsweise für Methyl oder Ethyl steht.

Europäisches Patentamt

# EUROPÄISCHER RECHERCHENBERICHT

Nummer der Anmeldung
EP 92 11 5158

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A<br><br>D | EP-A-0 433 749 (BAYER AG.)<br>* das ganze Dokument *<br>& DE-A-3 941 240<br>--- | 1,5,9 | C07D231/22<br>C07C69/72<br>A01N43/56 |
| A,D | EP-A-0 274 642 (BAYER AG)<br>* das ganze Dokument *<br>----- | 1,5,9 | |
| | | | RECHERCHIERTE SACHGEBIETE (Int. Cl.5 ) |
| | | | C07D<br>C07C |

Der vorliegende Recherchenbericht wurde für alle Patentansprüche erstellt

| Recherchenort | Abschlußdatum der Recherche | Prüfer |
|---|---|---|
| BERLIN | 04 JANUAR 1993 | KYRIAKAKOU G. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)